# EUROPEAN PATENT SPECIFICATION

(11) **EP 1 867 362 B1**
(45) Date of publication and mention of the grant of the patent: **13.04.2016**
(21) Application number: 06425341.2
(22) Date of filing: 16.06.2006
(51) Int. Cl.: A61N 1/378

(54) **Transcutaneous power supply with optimal positioning for use with active implantable devices**
Transkutane Energieversorgung mit optimaler Positionierung zur Verwendung in implantierbaren Geräten
Alimentation d'énergie transcutanée avec le positionnement optimal pour l'usage avec les dispositifs implantables actifs

(43) Date of publication of application: 19.12.2007
(73) Proprietor: Medico S.p.a., 35121 Padova (IT)
(72) Inventor: Mozzi, Aldo, c/o MEDICO S.p.a., 35121 Padova (IT); Snichelotto, Eugenio, c/o MEDICO S.p.a., 35121 Padova (IT)
(74) Representative: Vinci, Marcello

(56) References cited:
- WO-A-00/66221
- US-A- 5 314 453
- US-A- 5 948 006
- US-A1- 2006 129 205
- US-B1- 6 208 902

## Description

This patent relates to implantable medical devices and particularly concerns transcutaneous electrical power supply and/or recharging systems for such implantable devices.

In some electronic systems for implanting in the human body, it becomes necessary to supply power from an outside device to the implanted device (or implant).

Said power supply is normally provided by inductively coupling a coil called the transmitter and located outside the patient's body to a coil called the receiver, which is implanted just below the patient's skin and either connected to or incorporated in the implant.

The transmitter coil generates a field that is picked up and converted into electric current by the receiver coil when it is aligned with said transmitter coil.

The energy transferred to the implant can be exploited immediately or stored in a rechargeable battery for later use.

The outside device is hereinafter called the power supply, although it may equally serve as a battery recharger.

The effectiveness of the power transfer between the two coils is rather sensitive and depends mainly on their reciprocal positioning, i.e. on their coupling.

The best performance is obtained when the two coils, transmitter and receiver, are perfectly aligned, but this is not easy to achieve because it is difficult to identify the exact position of the receiver coil.

Any displacement or misalignment between the two coils reduces the energy transfer efficiency, with a smaller quantity of energy consequently being transferred to the implant, and/or increases the time needed to recharge the implant's battery.

Moreover, a part of the energy delivered by the transmitter coil that is not transferred to the receiver coil is absorbed by the tissues surrounding the coils, giving rise to an increase in the temperature of said tissues.

Given the time it takes the transmitter coil to recharge the implant's battery or to power the implant, exposing the tissues to the energy that is not absorbed by the receiver coil can have side effects of various type, ranging from mere discomfort to lesions with severe consequences.

There are also known electronic implantable devices that come complete with a telemetric intercommunication system to enable the transfer of information from the implant to other, outside devices, such as a programmer, a function monitor, a power supply, a recharger, and so on. Such telemetric systems are used to record and to set the implant's working parameters, to record historical data on how the implant functions, and other similar information.

Document US-A-2006/129205 discloses the most relevant prior art. The invention is defined in claim 1.

US A 2006/129205 *relates to a system for providing electrical pulses to cortical tissues of a patient comprising a implanted pulse generator, IPG, with an implantable coil, and external pulse generation means or programmer communicating with said IPG, with a external coil. In order to maximize the energy induced in the implanted coil by the external coil, said coils have to be property aligned. In this condition the voltage sensed by a voltage detector is at minimum level, whereas, when the coils become misaligned, the sensed voltage increases. The user, depending on the value of the voltage, can adjust the position of the external coil in respect of the implanted coill, to maximize the ener gy transferred.*

WO A 00/66221 *relates to an electromagnetic field source for providing electromagnetic energy to a secondary coil, comprising two or more primary coils and a controlled that selectively provides current to one or more of said primary coils, depending on their position with respect to the secondary coil.*

A new transcutaneous power supply and/or recharger for use with implants has been studied and developed to enable its precise positioning and centering over the receiver coil and thereby facilitate a better alignment between the transmitter coil and the receiver coil.

The object of the new power supply and/or recharger device complete with means for assisting in the device's positioning is to improve the positioning of the transmitter coil of the power supply device over the receiver coil of the device implanted in the patient's body by providing a quantitative indication of the power transfer being obtained at any given time.

Another object of the new power supply and/or recharger device is to facilitate the precise positioning of the transmitter coil of the power supply device over the receiver coil of the device implanted in the patient's body by showing the operator the direction in which the transmitter coil on the outside needs to be moved in order to be positioned precisely over the receiver coil.

Another object of the new power supply and/or recharger device is to facilitate the positioning of the transmitter coil of the power supply device over the receiver coil of the device implanted in the patient's body without the need for guide marks or reference points on the patient's body.

These and other, direct and complementary objects, are achieved through the implementation of the new transcutaneous power supply and/or recharger device for implants with means for assisting in its precise and centered positioning over the receiver coil of the implant, wherein the quantity of current received by the receiver coil is monitored by the implant and the power supply terminal of the power supply device is fitted with satellite sensors or coils arranged around the transmitter coil.

The recorded receiver coil voltage value can be sent to the power supply or recharger device via the existing telemetric channel, also indicating the quantity of the power transfer underway and thereby identifying the precision of the positioning between the power supply terminal and the receiver coil.

The power supply terminal's satellite coils or sensors (of which there are at least three, arranged around the transmitter coil) pick up signals and/or fields that depend on the transmitter coil and the metal bodies in the immediate vicinity.

At rest, the satellite sensors or coils pick up a signal and/or field due to the transmitter alone. When the transmitter coil is positioned perfectly over the receiver coil, the power supply terminal's satellite coils or sensors receive a different, weaker signal and/or field due to the presence of the receiver coil.

If the transmitter coil is not positioned perfectly over the receiver coil, however, the satellite coils or sensors receive a signal and/or field that is different from the one they receive when in the presence of the transmitter coil alone, and that depends on their distance from the receiver coil. Said difference in signal and/or field is recorded and used to provide a visual indication for the operator of the direction in which to move the power supply terminal and the corresponding transmitter coil in order to position it perfectly over the receiver coil.

The characteristics of the new transcutaneous power supply and/or recharger device for implants with means for assisting in its precisely centered positioning over the receiver coil of the implant will be better explained in the following description with reference to the attached drawings, which illustrate a non-restrictive example.

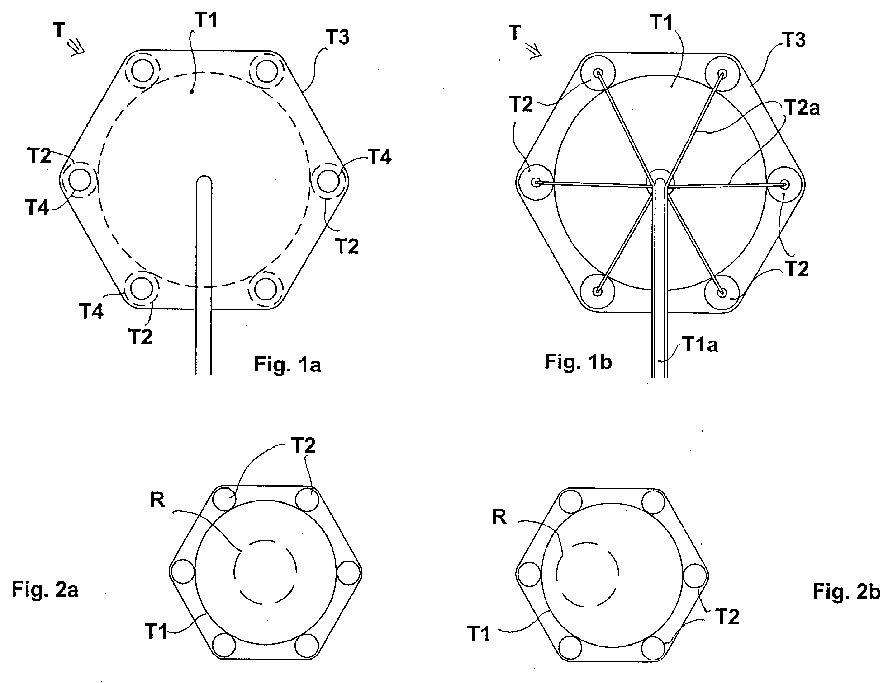

Figures 1a and 1b show an outer view and inner view, respectively, of an embodiment of the power supply terminal (T), comprising a holder (T3) containing a transmitter coil (T1) suitable for generating the power transmission field, and a number of smaller satellite coils or sensors (T2) arranged around said transmitter coil (T1).

The satellite coils or sensors (T2) may consist of small coils, possibly provided with a ferromagnetic core, or other types of sensor, e.g. *Hall* sensors.

In this example, to facilitate the description, reference is made to satellite coils (T2) consisting of coils with a ferromagnetic core.

The satellite coils (T2) lie on the same plane as the transmitter coil (T1).

These satellite coils (T2) can be arranged in any manner around the transmitter coil (T1), but are preferably placed equidistant from one another and also equidistant from the center of said transmitter coil (T1), i.e. in line with the vertices of a regular polygon concentric with the transmitter coil (T1).

One or more indicators (T4) are provided on the holder (T3) of the power supply terminal (T).

These indicators (T4) may consist, for instance, of a single display in a central position, or of various displays coinciding with the satellite coils (T2), or luminous indicators arranged in line with the satellite coils (T2), or analog indicators with dials placed in line with the satellite coils (T2).

The transmitter coil (T1) is connected to the power supply device (not shown) by means of a corresponding cable (T1a).

Each satellite coil (T2) is connected to a measurement and control circuit of the power supply device by means of a suitable cable (T2a).

Each satellite coil (T2) picks up a signal and/or field that depends on the transmitter coil (T1) and on any metal bodies in the vicinity.

At rest, the satellite coils (T2) perceive a signal and/or field that is due to the transmitter coil alone (T1). In this situation, the circuit controlling the power supply device can perform a calibration to determine the neutral position and the baseline reception of the satellite coils (T2), and can consequently represent the same value or information on the various indicators (T4) distributed in line with the various satellite coils (T2), or it can represent a centering signal on a single central indicator.

If the satellite coils (T2) are equidistant from one another and from the transmitter coil (T1), then all the satellite coils (T2) receive the same signal and/or field and all the indicators (T4) consequently provide the same indication.

When the transmitter terminal (T), and the transmitter coil (T1) in particular, are perfectly centered over the receiver coil (R), as illustrated in figure 2a, the satellite coils (T2) of the power supply terminal (T) all receive the same signal and/or field, which differs from the signal and/or field perceived in the presence of the transmitter coil (T1) alone.

Once the geoznetry of a given application has been defined, the trend of the fluxes in the satellite coils (T2) as a function of the position of the receiver coil (R) can be mapped and used for a more straightforward and/or precise calculation of the direction, or to eliminate the power supply calibration stage.

If the power supply terminal (T), and the transmitter coil (T1) in particular, are not positioned exactly over the receiver coil (R), as shown in the example in figure 2b, the satellite coils (T2) receive a different signal and/or field, which depends on their distance from the receiver coil (R) and also differs from the signal and/or field received in the presence of the transmitter coil alone (T1). Said difference in signal and/or field is recorded and compared by the power supply device's control circuit and used to provide a visual indication for the operator, by means of the indicators (T4), of the direction in which to move the power supply terminal (T) in order to position the transmitter coil (T1) exactly over the receiver coil (R).

By measuring the difference between the calibrated flux and the one actually picked up, the microprocessor incorporated in the power supply device can identify which of the satellite coils (T2) is nearest to the receiver coil (R) and thus indicate the direction in which to move the power supply terminal (T) in order to center it over the receiver coil (R). Using simple vector calculation methods, intermediate directions between two satellite coils (T2) can also be calculated.

The receiver coil (R) and implant continuously monitor the measurement of the voltage at the terminals of the receiver coil (R).

The voltage value recorded in the receiver coil (R) is sent by the implant to the power supply or recharger via the usual telemetric channel and indicates the quantity of the power transfer underway and thus the accuracy of the positioning of the power supply terminal (T) and receiver coil (R).

The new transcutaneous power supply and/or recharger device for implants with means for assisting in its accurately centered positioning over the receiver coil facilitates the precise positioning of the power supply terminal (T), or transmitter coil (T1), over the receiver coil (R) of the device implanted in the patient's body.

The new power supply and/or recharger device as described above facilitates a more accurate positioning of the transmitter coil (T1) over the receiver coil (R) of the device implanted in the patient's body by indicating to the operator the direction in which the power supply terminal (T), and thus the transmitter coil (T1), needs to be moved in order to obtain the perfect alignment between the transmitter coil (T1) and the receiver coil (T2).

The new power supply and/or recharger device as described above enables a constant monitoring of the efficiency of the power transfer from the transmitter coil (T1) to the receiver coil (R).

These are schematic, sufficient details for a person skilled in the art to implement the invention, but in practical application variants may be introduced without affecting the essence of the innovative concept.

Thus, with reference to the previous description and the attached drawings, the following claims are expressed.

## Claims

1. Transcutaneous power supply and/or recharger device for active implatable devices with a power supply terminal (T) comprising a holder (T3) containing a transmitter coil (T1) and optimal positioning means in order to optimize the energy transfer, wherein said optimal positioning means comprise at least three magnetic satellite sensors or coils (T2) arranged around the transmitter coil (T1) and suitable for picking up the signal and/or field generated by the transmitter coil (T1), which is modified by the presence, in the vicinity, of the implant's receiver coil (R), and wherein said power supply and/or recharger device comprises a control circuit connected to said coils or sensors (T2) for measuring and comparing the values and/or differences in the signal and/or field picked up by each of the satellite coils (T2), in order to indicate the direction in which the device needs to be moved, and wherein said device has one or more indicators (T4) situated on the power supply terminal (T), and wherein said indicators (T4) are differently energized or de-energized by the control circuit connected to the satellite sensors or coils (T2), depending on the signal and/or on a comparison of the differences in the signal and/or field recorded by said satellite coils (T2), enabling them to show the operator the direction in which to move the power supply terminal (T) and transmitter coil (T1) in order to position them optimally over the receiver coil (R).

2. Device according to claim 1, **characterized in that** said control circuit connected to said coils or sensors (T2) compares the values and/or differences in the signal and/or field picked up by each of the satellite coils (T2) with respect to a calibration signal acquired with the power supply terminal (T) with no metal objects in the vicinity.

3. Device according to claims 1, 2 **characterized in that** the position guiding system is used for the manual positioning of the device.

4. Device according to claims 1, 2, **characterized in that** the position guiding system is used for the automatic positioning of the device.

5. Transcutaneous power supply and/or recharger device according to the previous claims, **characterized in that** said control circuit connected to said satellite sensors or coils (T2) shows the operator the accurate alignment or the misalignment of the power supply terminal (T) and transmitter coil (T1) with respect to the receiver coil (R).

6. Transcutaneous power supply and/or recharger device according to the previous claims, **characterized in that** said satellite sensors or coils (T2) consist of electrical coils with a ferromagnetic core.

7. Transcutaneous power supply and/or recharger device according to the previous claims, **characterized in that** said satellite sensors or coils (T2) consist of sensors using semiconductor material.

8. Transcutaneous power supply and/or recharger device according to the previous claims, **characterized in that** the plane on which said satellite coils (T2) lie is substantially coplanar with the underside of the transmitter coil (T1).

9. Transcutaneous power supply and/or recharger device for implants according to claim 8, **characterized in that** the satellite coils (T2) are equidistant from the center of the transmitter coil (T1).

10. Transcutaneous power supply and/or recharger device for implants according to claim 8, **characterized in that** the satellite coils (T2) are arranged around the transmitter coil (T1) and equidistant from one another.

11. Transcutaneous power supply and/or recharger device for implants according to claim 1, **characterized in that** it has a single indicator (T4) consisting of a display, and wherein said control circuit connected to said satellite sensors or coils (T2) displays the direction in which to move the power supply terminal (T) in order to center the transmitter coil (T2) over the receiver coil (R).

12. Transcutaneous power supply and/or recharger device for implants according to claim 1, **characterized in that** it has an indictor (T4), preferably consisting of luminous indicators, or analog dial indicators, coinciding with each satellite sensor or coil (T2), and wherein said control circuit connected to the satellite sensors or coils (T2) disables or enables each of said indicators (T4) to indicate different distances, and thereby display the correct centering of the transmitter coil (T2) over the receiver coil (R).

13. Transcutaneous power supply and/or recharger device for implants according to the previous claims, **characterized in that** the implanted device is capable of continuously measuring the voltage at the terminals of the receiver coil (R), and wherein said voltage value recorded in the receiver coil (R) is sent to the power supply or recharger via a telemetric channel to indicate the efficacy of the positioning of the power supply terminal (T) and transmitter coil (T1) and the entity of the power transfer underway.

## Patentansprüche

1. Transkutane Stromversorgungs- und/oder Aufladevorrichtung für aktive, implantierbare Vorrichtungen mit einem Stromversorgungsanschluss (T), einen Halter (T3) umfassend, der eine Sendespule (T1) sowie Mittel zur optimalen Positionierung enthält, um die Energieübertragung zu optimieren, wobei die besagten Mittel zur optimalen Positionierung wenigstens drei magnetische Satellitensensoren oder -spulen ((T2) umfassen, die um die Sendespule (T1) herum angeordnet und geeignet sind, das durch die Sendespule (T1) erzeugte Signal und/oder Feld zu empfangen, welches durch die in der Nähe befindliche Empfängerspule (R) des Implantats verändert ist, und wobei die besagte Stromversorgungs- und/oder Aufladevorrichtung einen Steuerkreis umfasst, der an die besagten Spulen oder Sensoren (T2) angeschlossen sind, um die Werte und/oder Differenzen in dem durch jede der Satellitenspulen (T2) empfangenen Signal und/oder Feld zu messen und zu vergleichen, um die Richtung anzugeben, in welche die Vorrichtung bewegt werden muss, und wobei die besagte Vorrichtung eine oder mehrere Anzeigevorrichtungen (T4) am Stromversorgungsanschluss (T) aufweist, und wobei die besagten Anzeigevorrichtungen (T4) durch den an die Satellitensensoren oder -spulen (T2) angeschlossenen Steuerkreis je nach dem Signal und/oder einem Vergleich der Differenzen in dem durch die besagten Satellitenspulen (T2) aufgezeichneten Signal und/oder Feld unterschiedlich aktiviert oder deaktiviert werden, so dass sie dem Bediener die Richtung anzeigen können, in welche der Stromversorgungsanschluss (T) und die Sendespule (T1) verschoben werden müssen, um sie optimal über der Empfängerspule (R) zu positionieren.

2. Vorrichtung gemäß Patentanspruch 1, **dadurch gekennzeichnet, dass** der besagte, an die besagten Spulen oder Sensoren (T2) angeschlossene Steuerkreis die Werte und/oder Differenzen in dem durch jede der Satellitenspulen (T2) empfangenen Signal und/oder Feld bezüglich eines mit dem Stromversorgungsanschluss (T) erfassten Kalibrierungssignals vergleicht, wenn sich keine Metallobjekte in der Nähe befinden.

3. Vorrichtung gemäß den Patentansprüchen 1, 2, **dadurch gekennzeichnet, dass** das Positionsführungssystem zur manuellen Positionierung der Vorrichtung verwendet wird.

4. Vorrichtung gemäß den Patentansprüchen 1, 2, **dadurch gekennzeichnet, dass** das Positionsführungssystem zur automatischen Positionierung der Vorrichtung verwendet wird.

5. Transkutane Stromversorgungs- und/oder Aufladevorrichtung gemäß den vorstehenden Patentansprüchen, **dadurch gekennzeichnet, dass** der besagte, an die besagten Spulen oder Sensoren (T2) angeschlossene Steuerkreis dem Bediener die korrekte oder nicht korrekte Ausrichtung des Stromversorgungsanschlusses (T) und der Sendespule (T1) bezüglich der Empfängerspule (R) anzeigt.

6. Transkutane Stromversorgungs- und/oder Aufladevorrichtung gemäß den vorstehenden Patentansprüchen, **dadurch gekennzeichnet, dass** die besagten Satellitensensoren oder -spulen (T2) aus elektrischen Spulen mit einem ferromagnetischen Kern bestehen.

7. Transkutane Stromversorgungs- und/oder Aufladevorrichtung gemäß den vorstehenden Patentansprüchen, **dadurch gekennzeichnet, dass** die besagten Satellitensensoren oder -spulen (T2) aus Sensoren bestehen, die auf Halbleitermaterial basieren.

8. Transkutane Stromversorgungs- und/oder Aufladevorrichtung gemäß den vorstehenden Patentansprüchen, **dadurch gekennzeichnet, dass** die Ebene, auf der die besagten Satellitenspulen (T2) liegen, im Wesentlichen koplanar zu der Unterseite der Sendespule (T1) ist.

9. Transkutane Stromversorgungs- und/oder Aufladevorrichtung für Implantate gemäß Patentanspruch 8, **dadurch gekennzeichnet, dass** die Satellitenspulen (T2) gleich weit vom Mittelpunkt der Sendespule (T1) entfernt sind.

10. Transkutane Stromversorgungs- und/oder Aufladevorrichtung für Implantate gemäß Patentanspruch 8, **dadurch gekennzeichnet, dass** die Satellitenspulen (T2) um die Sendespule (T1) herum angeordnet und gleich weit voneinander entfernt sind.

11. Transkutane Stromversorgungs- und/oder Aufladevorrichtung für Implantate gemäß Patentanspruch 1, **dadurch gekennzeichnet, dass** sie eine einzige Anzeigevorrichtung (T4) aufweist, die aus einem Display besteht, und wobei der besagte, an die besagten Satellitensensoren oder Spulen (T2) angeschlossene Steuerkreis die Richtung anzeigt, in welche der Stromversorgungsanschluss (T) bewegt werden muss, um die Sendespule (T2) über der Empfängerspule (R) zu zentrieren.

12. Transkutane Stromversorgungs- und/oder Aufladevorrichtung für Implantate gemäß Patentanspruch 1, **dadurch gekennzeichnet, dass** sie eine Anzeigevorrichtung (T4) aufweist, die vorzugsweise aus Leuchtanzeigen oder Analog-Messuhren besteht, welche mit jedem Satellitensensor oder jeder Spule (T2) zusammenfallen, und wobei der besagte, an die besagten Satellitensensoren oder Spulen (T2) angeschlossene Steuerkreis jede der besagten Anzeigevorrichtungen (T4) aktiviert oder deaktiviert, um unterschiedliche Entfernungen anzuzeigen, und dadurch die korrekte Zentrierung der Sendespule (T2) über der Empfängerspule (R) anzuzeigen.

13. Transkutane Stromversorgungs- und/oder Aufladevorrichtung für Implantate gemäß den vorstehenden Patentansprüchen, **dadurch gekennzeichnet, dass** die implantierte Vorrichtung in der Lage ist, die Spannung an den Anschlüssen der Empfängerspule (R) kontinuierlich zu messen, und wobei der besagte, in der Empfängerspule (R) aufgezeichnete Spannungswert über einen telemetrischen Kanal an die Stromversorgung oder die Aufladevorrichtung gesendet wird, um die Wirksamkeit der Positionierung des Stromversorgungsanschlusses (T) und der Sendespule (T1) sowie den Umfang der laufenden Energieübertragung anzuzeigen.

## Revendications

1. Dispositif d'alimentation et/ou de recharge transcutané pour dispositifs implantables actifs, ayant un terminal d'alimentation (T) comprenant un support (T3) contenant une bobine émettrice (T1) et des moyens de positionnement optimaux de façon à optimiser le transfert d'énergie, où lesdits moyens de positionnement optimaux comprennent au moins trois capteurs ou bobines satellites magnétiques (T2) disposés autour de la bobine émettrice (T1) et aptes à capter le signal et/ou le champ généré par la bobine émettrice (T1), qui est modifié par la présence, à proximité, de la bobine réceptrice (R) du dispositif implanté, et où ledit dispositif d'alimentation et/ou de recharge comprend un circuit de contrôle relié auxdites bobines ou capteurs (T2), apte à mesurer et comparer les valeurs et/ou les différences de signal et/ou de champ capté par chacune desdites bobines satellites (T2), de manière à indiquer la direction dans laquelle il est nécessaire de déplacer le dispositif, et où ledit dispositif présente un ou plusieurs indicateurs (T4) disposés sur le terminal d'alimentation (T), et où lesdits indicateurs (T4) sont différemment activés ou désactivés par le circuit de contrôle aux capteurs ou bobines satellites (T2), selon le signal et/ou une comparaison des différences dans le signal et/ou le champ enregistré par lesdites bobines satellites (T2), en leur permettant de montrer à l'opérateur la direction dans laquelle on peut déplacer le terminal d'alimentation (T) et la bobine émettrice (T1) de façon à les positionner de manière optimale sur la bobine de réceptrice (R).

2. Dispositif selon la revendication 1, **caractérisé en ce que** ledit circuit de contrôle relié auxdites bobines ou capteurs (T2) compare les valeurs et/ou les différences dans le signal et/ou le champ captées par chacune des bobines satellites (T2) par rapport à un signal de calibrage acquis avec le terminal d'alimentation (T) avec aucun objet métallique à proximité.

3. Dispositif selon les revendications 1 et 2, **caractérisé en ce que** le système de guidage de postion est utilisé pour le positionnement manuel du dispositif

4. Dispositif selon les revendications 1 et 2, **caractérisé en ce que** le système de guidage de position est utilisé pour le positionnement automatique du dispositif.

5. Dispositif d'alimentation et/ou de recharge transcutané selon les revendications précédentes, **caractérisé en ce que** ledit circuit de contrôle relié auxdits capteurs ou bobines satellites (T2) montre à l'opérateur l'alignement ou le désalignement précis du terminal d'alimentation (T) et de la bobine émettrice (T1) par rapport à la bobine réceptrice (R).

6. Dispositif d'alimentation et/ou de recharge transcutané selon les revendications précédentes, **caractérisé en ce que** lesdits capteurs ou bobines satellites (T2) se composent de bobines électriques avec noyau ferromagnétique.

7. Dispositif d'alimentation et/ou de recharge transcutané selon les revendications précédentes, **caractérisé en ce que** lesdits capteurs ou bobines satellites (T2) se composent de capteurs utilisant un matériau semi-conducteur.

8. Dispositif d'alimentation et/ou de recharge transcutané selon les revendications précédentes, **caractérisé en ce que** le plan sur lequel lesdites bobines satellites (T2) reposent est essentiellement coplanaire avec le dessous de la bobine émettrice (T1).

9. Dispositif d'alimentation et/ou de recharge transcutané pour implants selon la revendication 8, **caractérisé en ce que** les bobine satellites (T2) sont équidistantes du centre de la bobine émettrice (T1).

10. Dispositif d'alimentation et/ou de recharge transcutané pour implants selon la revendication 8, **caractérisé en ce que** les bobines satellites (T2) sont disposées autour de la bobine émettrice (T1) et équidistantes entre elles.

11. Dispositif d'alimentation et/ou de recharge transcutané pour implants selon la revendication 1, **caractérisé en ce qu'**il présente un seul indicateur (T4) se composant d'un afficheur, et où ledit circuit de contrôle relié auxdits capteurs ou bobines satellites (T2) affiche la direction dans laquelle le terminal d'alimentation (T) se déplace afin de pouvoir centrer la bobine émettrice (T2) sur la bobine réceptrice (R).

12. Dispositif d'alimentation et/ou de recharge transcutané pour implants selon la revendication 1, **caractérisé en ce qu'**il présente un indicateur (T4), préférablement se composant d'indicateurs lumineux, ou indicateurs à cadran analogique, coïncidant avec chaque capteur ou bobine satellite (T2), et où ledit circuit de contrôle relié aux capteurs ou bobines satellites (T2) désactive ou active chacun desdits indicateurs (T4) afin d'indiquer des distances différentes, et donc d'afficher le centrage correct de la bobine émettrice (T2) sur la bobine réceptrice (R).

13. Dispositif d'alimentation et/ou de recharge transcutané pour implants selon les revendications précédentes, **caractérisé en ce que** le dispositif implanté est en mesure de mesurer constamment la tension sur les terminaux de la bobine réceptrice (R), et où ladite valeur de tension enregistrée dans la bobine réceptrice (R) est envoyée à l'alimentateur ou chargeur par un canal télémétrique pour indiquer l'efficacité du positionnement du terminal d'alimentation (T) et de la bobine émettrice (T1) et l'entité du transfert d'alimentation en cours.
